# EUROPEAN PATENT SPECIFICATION

(11) **EP 3 360 867 B1**
(45) Date of publication and mention of the grant of the patent: **01.09.2021**
(21) Application number: 15905712.4
(22) Date of filing: 02.11.2015
(51) Int. Cl.: C07D 277/72

(54) **PREPARATION METHOD OF 2-MERCAPTOBENZOTHIAZOLE**
HERSTELLUNGSVERFAHREN FÜR 2-MERCAPTOBENZOTHIAZOL
PROCÉDÉ DE PRÉPARATION DE 2-MERCAPTOBENZOTHIAZOLE

(30) Priority: 09.10.2015 CN 201510649388
(43) Date of publication of application: 15.08.2018
(73) Proprietor: Sennics Co., Ltd., Pudong New Area Shanghai 200126 (CN)
(72) Inventor: CHEN, Xinmin, Shanghai 200126 (CN); WU, Libao, Shanghai 200126 (CN); SHI, Song, Shanghai 200126 (CN); GAO, Shang, Shanghai 200126 (CN)
(74) Representative: Santi, Filippo
(86) International application number: PCT/CN2015/093613
(87) International publication number: WO 2017/059614

(56) References cited:
- CN-A- 101 508 675
- CN-A- 102 070 562
- CN-A- 102 304 099
- ZHU, QIANG ET AL.: 'Progress of Sulfonic Acid Functional Ionic Liquids in Organic Synthesis' CHEMICAL PRODUCTION AND TECHNOLOGY vol. 18, no. 2, 31 December 2011, pages 38 - 44, XP009505625
- LIU, RUIJIANG ET AL.: 'Optimization of Synthesis of 2-Mercapto Benzothiazole with Response Surface Methodology' CHEMICAL INDUSTRY AND ENGINEERING PROGRESS vol. 28, no. 1, 31 December 2009, pages 155 - 158 AND 172, XP009505584

## Description

### Technical Field

The disclosure relates to the field of organic synthesis, and particularly to a preparation method for 2-mercaptobenzothiazole.

### Background

2-mercaptobenzothiazole (also called as an accelerator M or an accelerator MBT and further called as quick heating powder) is a general-purpose rubber vulcanization accelerator. It has the characteristics of fast vulcanization acceleration action, low vulcanization flatness, no early vulcanization during mixing and the like, and is widely applied to the rubber processing industry.

There are many synthesis methods for the accelerator M, and according to adopted raw materials, may be divided into an o-nitrochlorobenzene method, an aniline method, a mixed nitrobenzene and aniline method, a nitrobenzene method, a nitroso-benzene method and the like. The o-nitrochlorobenzene method, the aniline method and the mixed nitrobenzene and aniline method are usually adopted more. However, when the o-nitrochlorobenzene method is adopted for producing the accelerator M, cost of raw materials is high, and a production process is complex, so that it is unsuitable for industrial application. The mixed nitrobenzene and aniline method is low in production cost, and may reduce H₂S produced by reaction by 1/3 of the aniline method, but it has the problems of difficulties in control of the reaction and high requirements on material of reactor, so that the method is adopted less in China at present. Therefore, the aniline method for synthesizing the accelerator M is a method generally adopted by each auxiliary producer.

CN102 070 562A discloses reacting carbon disulfide, aniline and sulfur in a molar ratio of 1.0-1.5 : 1: 1.5-2.5, at a reaction temperature of 240° C-260° C and a reaction pressure of 6.8-9.5 MPa for 80-120 min; and performing, in a crystallizer, treatments, such as cooling, on the resulting crude product melt so as to obtain a 2-mercaptobenzothiazole with a mass percentage content of greater than 98%. The reaction can be recycled, and the total reaction yield of the product is over 94%.

CN102 304 099A discloses reacting an aniline derivative, carbon disulfide, and sulfur in a molar ratio of 1 : 1.0-2.0 : 1.0-3.0 at a reaction temperature of 200° C-260° C and a reaction pressure of 6-15 MPa for a reaction time of 2-5 h to prepare a 2-mercaptobenzothiazole derivative.The product is dissolved in an alkali, and the side reaction impurities are removed by filtration, and the content of the acidified precipitated product is 99% or more, and the reation yield can be increased to 90% to 100% by optimizing the reaction conditions.

The aniline method for producing the accelerator M has the characteristics of stable raw material sources, low difficulties in operation and low requirements on the material of reactor. However, it still has the shortcomings of relatively low purity of a product, high tar yield and relatively low yield.

### Summary

The disclosure is intended to provide a preparation method for 2-mercaptobenzothiazole, so as to solve the problems of relatively low purity of a product, high tar yield and relatively low yield when an accelerator M is produced by an aniline method in prior art.

In order to achieve the purpose, according to an aspect of the disclosure, a preparation method for 2-mercaptobenzothiazole is provided, which includes that: an aniline method is adopted to perform reaction in the presence of a catalyst to prepare the 2-mercaptobenzothiazole, the catalyst comprising sulfonic acid type imidazolium ionic liquid.

Furthermore, the sulfonic acid type imidazolium ionic liquid is [TSVM]HSO₄ and/or [MPS]₂HSO₄.

Furthermore, the catalyst further includes a carrier for supporting the sulfonic acid type imidazolium ionic liquid.

Furthermore, the carrier is selected from one or more of a group formed by an activated alumina, cordierite honeycomb ceramics, mercaptoalkyl-functionalised silica gel, an ion exchange resin and a HZSM molecular sieve (Hydrogen type Zeolite Sieve of Molecular porosity molecular sieve).

Furthermore, a weight ratio of the sulfonic acid type imidazolium ionic liquid and the carrier is (0.1 ∼20):100.

Furthermore, a reaction temperature of the reaction is 200∼280 °C, reaction pressure is 3.0∼8.0MPa, and reaction time is 2∼12h.

Furthermore, the reaction temperature of the reaction is 240∼250°C, the reaction pressure is 4.5∼5.5MPa, and the reaction time is 4∼6h.

Furthermore, in the aniline method, an aniline, a carbon disulfide and a sulfur are taken as reaction raw materials, a molar ratio of the aniline, the carbon disulfide and the sulfur is 1:(1.0∼1.3):(1.1∼2), and a weight ratio of the aniline and the sulfonic acid type imidazolium ionic liquid is 100:(1∼5).

Furthermore, the molar ratio of the aniline, the carbon disulfide and the sulfur is 1:(1.0∼1.1):(1.2∼1.5), and the weight ratio of the aniline and the sulfonic acid type imidazolium ionic liquid is 100:(1∼2).

Furthermore, the 2-mercaptobenzothiazole is prepared by adopting an intermittent or continuous production process.

In the preparation method provided by the disclosure, the aniline method is adopted to prepare the 2-mercaptobenzothiazole with a catalytic action of the sulfonic acid type imidazolium ionic liquid. The sulfonic acid type imidazolium ionic liquid is a type of acidic functionalized ionic liquid, and has the advantages of both a solid acid and a liquid acid. In the disclosure, the sulfonic acid type imidazolium ionic liquid is adopted as an active ingredient of the catalyst, and may remarkably improve a conversion rate of the reaction raw materials and increase a yield of the 2-mercaptobenzothiazole. Meanwhile, due to the characteristics of high catalytic activity, no volatilization, low corrosion and high thermal stability of the 2-mercaptobenzothiazole, the preparation method provided by the disclosure also has the comprehensive advantages of simple process, low cost, low tar yield and high environment friendliness.

### Detailed Description of the Embodiments

As described in the Background, when 2-mercaptobenzothiazole is produced by a prior aniline method, there exist the problems of relatively low purity of a product, high tar yield and relatively low yield. In order to solve the problems, the inventor of the disclosure provides a preparation method for 2-mercaptobenzothiazole. An aniline method is adopted to perform reaction in the presence of a catalyst to prepare the 2-mercaptobenzothiazole, and the catalyst includes sulfonic acid type imidazolium ionic liquid.

In the preparation method provided by the disclosure, the aniline method is adopted to prepare the 2-mercaptobenzothiazole under a catalytic action of the sulfonic acid type imidazolium ionic liquid. The sulfonic acid type imidazolium ionic liquid is a type of acidic functionalized ionic liquid, and has the advantages of both a solid acid and a liquid acid. In the disclosure, the sulfonic acid type imidazolium ionic liquid is adopted as an active ingredient of the catalyst, and may remarkably improve a conversion rate of the reaction raw materials and increase a yield and a purity of the 2-mercaptobenzothiazole. Meanwhile, due to the characteristics of high catalytic activity, no volatilization, low corrosion and high thermal stability of the 2-mercaptobenzothiazole, the preparation method provided by the disclosure also has the comprehensive advantages of simple process, low cost, low tar yield and high environment friendliness.

Compared with CN102 070 562A and CN102 304 099A, the distinguishing of the preparation method according to the present invention is the introduction of a sulfonic acid type imidazolium ionic liquid as an active ingredient of the catalyst.

In the preparation method provided by the disclosure, as long as the sulfonic acid type imidazolium ionic liquid is adopted to catalyze the reaction between aniline, carbon disulfide and sulfur as the active ingredient, a reaction process may be endowed with the advantages of high yield, low cost and environment friendliness. In a preferred embodiment, the sulfonic acid type imidazolium ionic liquid is [TSVM]HSO₄ and/or [MPS]₂HSO₄.

Wherein, a structure of the [TSVM]HSO₄ ionic liquid is as follows: 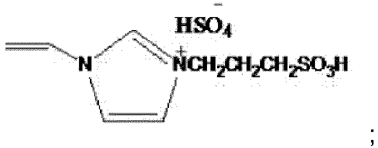 a structure of the [MPS]₂HSO₄ ionic liquid is as follows:

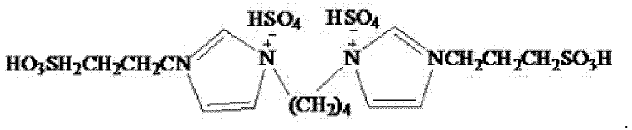

Both of the two types of sulfonic acid type imidazolium ionic liquid have relatively high catalytic activity, and are relatively low in cost and extensive in source, and using them as the active ingredient of the catalyst may further increase the yield of the 2-mercaptobenzothiazole, reduce emissions of waste gas, waste water and sold waste and reduce production cost.

In the preparation method of the disclosure, the sulfonic acid type imidazolium ionic liquid may be directly adopted to catalytically prepare the 2-mercaptobenzothiazole as a catalyst, that is, non-supported sulfonic acid type imidazolium ionic liquid may be directly added into a reaction system for catalytic reaction. In a preferred embodiment, a supported sulfonic acid type imidazolium ionic liquid is adopted as a catalyst, that is, the catalyst further includes a carrier for supporting the sulfonic acid type imidazolium ionic liquid. The supported sulfonic acid type imidazolium ionic liquid catalyst not only is applied to catalytic reaction in an ordinary intermittent reaction kettle or kettle type continuous reactor, but also may be mounted in a fixed tower to realize continuous production by continuous input and output of reaction raw materials, and has the advantages of relatively high yield, relatively low energy consumption and relatively low production cost.

According to the abovementioned instruction of the disclosure, those skilled in the art may select a concrete carrier of the supported sulfonic acid type imidazolium ionic liquid catalyst. In a preferred embodiment, the carrier includes, but not limited to, one or more of an active alumina, a cordierite honeycomb ceramics, a mercaptoalkyl-functionalised silica gel, an ion exchange resin and an HZSM molecular sieve. All of these carriers have the advantages of stable physical properties, large specific surface area and relatively better bonding action with the sulfonic acid type imidazolium ionic liquid. In addition, these carriers are also relatively low in cost.

In the supported catalyst, those skilled in the art may select a proportional relationship between the catalytic active ingredient and the carrier. In a preferred embodiment, a weight ratio of the sulfonic acid type imidazolium ionic liquid and the carrier is (0.1 ∼20):100. A relationship between using amounts of the two is set within the abovementioned range, so that the active ingredient of the catalyst may be dispersed on the carrier more uniformly, and relatively high catalytic activity is achieved. Meanwhile, cost may also be reduced, and difficulties in reaction operations may be reduced.

In a preferred embodiment, a reaction temperature of the reaction in a preparation process is 200∼280°C, reaction pressure is 3.0∼8.0MPa, and reaction time is 2∼12h. In the preparation method provided by the disclosure, the sulfonic acid type imidazolium ionic liquid is adopted as the catalytic active ingredient, so that difficulties in operations for the 2-mercaptobenzothiazole may be reduced, and reaction conditions may be milder. The temperature, the pressure and the time conditions are favorable for increasing a reaction rate, simultaneously reducing occurrence of side reaction and endowing the reaction with a relatively high rate and yield. More preferably, the reaction temperature of the reaction is 240∼250°C, the reaction pressure is 4.5∼5.5MPa, and the reaction time is 4∼6h.

In the preparation method provided by the disclosure, those skilled in the art may select a relationship between using amounts of each raw material. In a preferred embodiment, in the aniline method, an aniline, a carbon disulfide and a sulfur are taken as reaction raw materials, a molar ratio of the aniline, the carbon disulfide and the sulfur is 1:(1.0∼1.3):(1.1∼2), and a weight ratio of the aniline and the sulfonic acid type imidazolium ionic liquid is 100:(1∼5). Setting the ratio of the using amounts of each raw material within the range is favorable for further increasing the conversion rate of the reaction and simultaneously achieving a higher reaction speed and a higher target product yield. In addition, controlling the using amount of the catalyst within the abovementioned range is also favorable for reducing the cost and making the whole reaction more applicable to industrial large-scale application. More preferably, the molar ratio of the aniline, the carbon disulfide and the sulfur is 1:(1.0∼1.1):(1.2∼1.5), and the weight ratio of the aniline and the sulfonic acid type imidazolium ionic liquid is 100:(1∼2).

The preparation method provided by the disclosure is applied to any production mode. In a preferred embodiment, the 2-mercaptobenzothiazole is prepared by adopting an intermittent or continuous production process. Intermittent refers to an intermittent kettle type catalytic reaction manner, and continuous refers to a continuous tower type reaction manner (for example, a fixed bed reaction manner or a trickle bed reaction manner).

The disclosure will further be described below in combination with example s in detail, and these embodiments should not be understood to limit the scope of protection of the disclosure.

### Example 1

93 grams of an aniline, 76 grams of a carbon disulfide and 38.4 grams of a sulfur (a molar ratio of the three is 1:1:1.2) were added into a pressure reactor, and 1.86 grams of a non-supported sulfonic acid type imidazolium ionic liquid [TSVM]HSO₄ (a weight ratio between the aniline and it is 100:2) was added into a system. A reaction temperature was controlled to be 250°C, a reaction pressure was controlled to be 4.5MPa, and after 6h reaction, a crude product melt was obtained.

The crude product melt was pressed into a crystallizer containing a toluene solvent, discharged hydrogen sulfide was absorbed and treated with alkali liquor, and after the melt was crystalized, washing, filtering and drying were performed to obtain a 2-mercaptobenzothiazole product, wherein the product has a purity 98% or more and a yield of 90%.

### Examples 2 to 7

A preparation method was the same as example 1, and differences were in the reaction temperatures, the reaction pressure and the reaction time, and process conditions and product results of each example are shown in the following table.

| | Reaction temperature (°C) | Reaction pressure (MPa) | Reaction time (h) | Product purity (%) | Product yield (%) |
|---|---|---|---|---|---|
| Example 2 | 240 | 5.5 | 6 | 98 | 92 |
| Example 3 | 240 | 4.0 | 4 | 97.5 | 92 |
| Example 4 | 200 | 8.0 | 12 | 97 | 84 |
| Example 5 | 200 | 8.0 | 14 | 97.2 | 83 |
| Example 6 | 280 | 3.0 | 2 | 95.4 | 84 |
| Example 7 | 300 | 10.0 | 2 | 96.3 | 83 |

### Examples 8 to 13

A preparation method was the same as embodiment 1, and differences were in the molar ratios of each raw material, and raw material relationships and product results of each example are shown in the following table.

| | Molar ratio of aniline, carbon disulfide and sulfur | Weight ratio of aniline and [TSVM]HSO₄ | Product purity (%) | Product yield (%) |
|---|---|---|---|---|
| Example 8 | 1:1.1:1.5 | 100:2 | 98 | 90 |
| Example 9 | 1:1.1:1.5 | 100:1 | 98 | 89 |
| Example 10 | 1:1.1:1.1 | 100:3 | 97 | 91 |
| Example 11 | 1:1.1:2 | 100:4 | 97 | 92 |
| Example 12 | 1:1.3:2 | 100:5 | 98 | 90 |
| Example 13 | 1:1:1 | 100:10 | 98 | 92 |

### Examples 14 to 19

A preparation method was the same as example 1, and differences were in types of the catalyst, and catalyst types and product results of each example are shown in the following table.

| | Catalyst | | | | Pr oduct purity (%) | Pr oduct yield (%) |
|---|---|---|---|---|---|---|
| | Sulfonic acid type imidazolium ionic liquid | | Carrier | | | |
| | Type | Weight (g) | Type | We ight (g) | | |
| Example 14 | [TSVM]HSO₄ | 1.86 | HZSM molar sieve | 9.3 | 97 | 90 |
| Example 15 | [TSVM]HSO₄ | 1.86 | HZSM molar sieve | 18. 6 | 98 | 91 |
| Example 16 | [TSVM]HSO₄ | 0.37 | HZSM molar sieve | 37 0 | 94 | 87 |
| Example 17 | [MPS]₂HSO₄ | 1.86 | NONE | | 96 | 91 |
| Example 18 | [MPS]₂HSO₄ | 1.86 | cordierite honeycomb ceramics | 6.2 | 98 | 92 |
| Example 19 | [TSVM]HSO₄ /[MPS]₂HSO₄ (weight ratio: 1:1) | 1.86 | mercapto alkyl-function alised silica gel | 7.4 | 98 | 90 |

From the above descriptions, it can be seen that the examples of the disclosure achieve the following technical effects: the sulfonic acid type imidazolium ionic liquid is adopted as the active ingredient of the catalyst, and may remarkably improve a conversion rate of the reaction raw materials and increase a yield of the 2-mercaptobenzothiazole. Meanwhile, the preparation method provided by the disclosure also has the comprehensive advantages of simple process, low cost, low tar yield and high environment friendliness.

## Claims

1. A preparation method for 2-mercaptobenzothiazole, wherein an aniline method is adopted to perform reaction in the presence of a catalyst to prepare the 2-mercaptobenzothiazole, the catalyst comprising sulfonic acid type imidazolium ionic liquid.

2. The preparation method as claimed in claim 1, wherein the sulfonic acid type imidazolium ionic liquid is 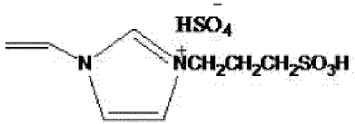 and/or

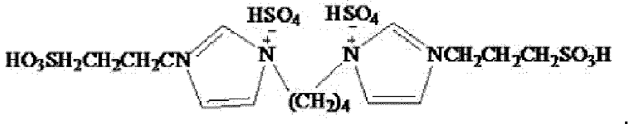

3. The preparation method as claimed in claim 1 or 2, wherein the catalyst further comprises a carrier for supporting the sulfonic acid type imidazolium ionic liquid.

4. The preparation method as claimed in claim 3, wherein the carrier is selected from one or more of a group formed by an activated alumina, a cordierite honeycomb ceramics, a mercaptoalkyl-functionalised silica gel, an ion exchange resin and a HZSM molecular sieve.

5. The preparation method as claimed in claim 4, wherein a weight ratio of the sulfonic acid type imidazolium ionic liquid and the carrier is (0.1∼20):100.

6. The preparation method as claimed in claim 4 or 5, wherein a reaction temperature of the reaction is 200∼280°C, reaction pressure is 3.0∼8.0MPa, and reaction time is 2∼12h.

7. The preparation method as claimed in claim 6, wherein the reaction temperature of the reaction is 240∼250°C, the reaction pressure is 4.5∼5.5MPa, and the reaction time is 4∼6h.

8. The preparation method as claimed in claim 1, wherein, in the aniline method, an aniline, a carbon disulfide and a sulfur are taken as reaction raw materials, a molar ratio of the aniline, the carbon disulfide and the sulfur is 1:(1.0∼1.3):(1.1∼2), and a weight ratio of the aniline and the sulfonic acid type imidazolium ionic liquid is 100:(1∼5).

9. The preparation method as claimed in claim 8, wherein the molar ratio of the aniline, the carbon disulfide and the sulfur is 1:(1.0∼1.1):(1.2∼1.5), and the weight ratio of the aniline and the sulfonic acid type imidazolium ionic liquid is 100:(1∼2).

10. The preparation method as claimed in claim 1, wherein the 2-mercaptobenzothiazole is prepared by adopting an intermittent or continuous production process.

## Patentansprüche

1. Herstellungsverfahren für 2-Mercaptobenzothiazol, wobei ein Anilinverfahren angewendet wird, um die Reaktion in Gegenwart eines Katalysators durchzuführen, um das 2-Mercaptobenzothiazol herzustellen, wobei der Katalysator eine Sulfonsäuretyp-Imidazolium-Ionenflüssigkeit umfasst.

2. Herstellungsverfahren nach Anspruch 1, wobei die Sulfonsäuretyp-Imidazolium-Ionenflüssigkeit 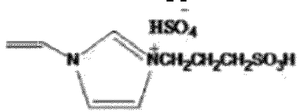 ist, und/oder

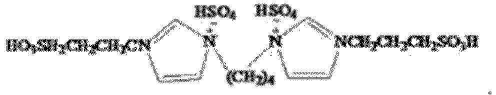

3. Herstellungsverfahren nach Anspruch 1 oder 2, wobei der Katalysator ferner einen Träger zum Tragen der Sulfonsäuretyp-Imidazolium-Ionenflüssigkeit umfasst.

4. Herstellungsverfahren nach Anspruch 3, wobei der Träger aus einem oder mehreren einer Gruppe ausgewählt ist, die aus einem aktivierten Aluminiumoxid, einer Cordierit-Wabenkeramik, einem Mercaptoalkylfunktionalisierten Silicagel, einem Ionenaustauscherharz und einem HZSM-Molekularsieb besteht.

5. Herstellungsverfahren nach Anspruch 4, wobei das Gewichtsverhältnis der Sulfonsäuretyp-Imidazolium-Ionenflüssigkeit und des Trägers (0,1∼20):100 beträgt.

6. Herstellungsverfahren nach Anspruch 4 oder 5, wobei die Reaktionstemperatur der Reaktion 200∼280°C beträgt, der Reaktionsdruck 3,0∼8,0 MPa beträgt und die Reaktionszeit 2∼12h beträgt.

7. Herstellungsverfahren nach Anspruch 6, wobei die Reaktionstemperatur der Reaktion 240∼250°C beträgt, der Reaktionsdruck 4,5∼5,5 MPa beträgt und die Reaktionszeit 4∼6h beträgt.

8. Herstellungsverfahren nach Anspruch 1, wobei bei dem Anilinverfahren ein Anilin, ein Kohlenstoffdisulfid und ein Schwefel als Reaktionsrohstoffe verwendet werden, wobei ein Molverhältnis von Anilin, Schwefelkohlenstoff und Schwefel 1:(1,0∼1,3):(1,1∼2) beträgt, und das Gewichtsverhältnis des Anilins und der Sulfonsäuretyp-Imidazolium-Ionenflüssigkeit 100:(1∼5) beträgt.

9. Herstellungsverfahren nach Anspruch 8, wobei das Molverhältnis des Anilins, des Kohlenstoffdisulfids und des Schwefels 1:(1,0∼1,1):(1,2∼1,5) beträgt und das Gewichtsverhältnis des Anilins und der Sulfonsäuretyp-Imidazolium-Ionenflüssigkeit 100:(1∼2) beträgt.

10. Herstellungsverfahren nach Anspruch 1, wobei das 2-Mercaptobenzothiazol durch Anwenden eines intermittierenden oder kontinuierlichen Herstellungsverfahrens hergestellt wird.

## Revendications

1. Procédé de préparation du 2-mercaptobenzothiazole, dans lequel un procédé à l'aniline est adopté pour effectuer une réaction en présence d'un catalyseur pour préparer le 2-mercaptobenzothiazole, le catalyseur comprenant un liquide ionique d'imidazolium de type acide sulfonique.

2. Procédé de préparation selon la revendication 1, dans lequel le liquide ionique d'imidazolium de type acide sulfonique est 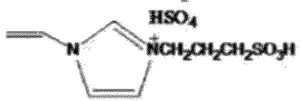 et/ou

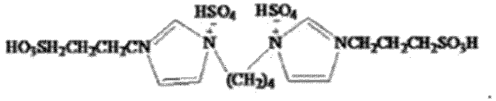

3. Procédé de préparation selon la revendication 1 ou 2, dans lequel le catalyseur comprend en outre un support pour supporter le liquide ionique d'imidazolium de type acide sulfonique.

4. Procédé de préparation selon la revendication 3, dans lequel le support est choisi parmi un ou plusieurs d'un groupe formé par une alumine activée, une céramique en nid d'abeille de cordiérite, un gel de silice à fonction mercaptoalkyle, une résine échangeuse d'ions et un tamis moléculaire HZSM.

5. Procédé de préparation selon la revendication 4, dans lequel un rapport pondéral du liquide ionique d'imidazolium de type acide sulfonique et du support est (0,1∼20):100.

6. Procédé de préparation selon la revendication 4 ou 5, dans lequel une température de réaction de la réaction est de 200∼280°C, la pression de réaction est de 3,0∼8,0 MPa, et le temps de réaction est de 2∼12 h.

7. Procédé de préparation selon la revendication 6, dans lequel la température de réaction de la réaction est de 240∼250°C, la pression de réaction est de 4,5-5,5 MPa, et le temps de réaction est de 4∼6 h.

8. Procédé de préparation selon la revendication 1, dans lequel, dans le procédé à l'aniline, on prend comme matières premières réactionnelles une aniline, un disulfure de carbone et un soufre, un rapport molaire de l'aniline, du disulfure de carbone et du soufre est de 1:(1,0∼1,3):(1,1∼2), et un rapport pondéral de l'aniline et du liquide ionique d'imidazolium de type acide sulfonique est de 100:(1∼5).

9. Procédé de préparation selon la revendication 8, dans lequel le rapport molaire de l'aniline, du disulfure de carbone et du soufre est de 1:(1,0∼1,1):(1,2∼1,5), et le rapport pondéral de l'aniline et du liquide ionique d'imidazolium de type acide sulfonique est de 100:(1∼2).

10. Procédé de préparation selon la revendication 1, dans lequel le 2-mercaptobenzothiazole est préparé en adoptant un procédé de production intermittent ou continu.
